# EUROPEAN PATENT SPECIFICATION

(11) **EP 3 909 576 B1**
(45) Date of publication and mention of the grant of the patent: **15.11.2023**
(21) Application number: 20738511.3
(22) Date of filing: 10.01.2020
(51) Int. Cl.: A61K 31/216, A61P 25/00, A61K 9/08, A61K 9/20, A61P 29/00, A61P 19/02, A61P 21/00, A61K 9/00

(54) **CHLOROGENIC ACID FOR USE IN THE TREATMENT OF CANCER PAIN**
CHLOROGENSÄURE ZUR VERWENDUNG BEI DER BEHANDLUNG VON KREBSSCHMERZEN
ACIDE CHLOROGÉNIQUE POUR UTILISATION DANS LE TRAITEMENT DE LA DOULEUR CANCÉREUSE

(30) Priority: 11.01.2019 CN 201910029815
(43) Date of publication of application: 17.11.2021
(73) Proprietor: Sichuan Jiuzhang Biological Science And Technology Co., Ltd, High-tech District Chengdu Sichuan 610041 (CN)
(72) Inventor: ZHANG, Jie, Chengdu, Sichuan 610041 (CN); ZHANG, Yazhuo, Chengdu, Sichuan 610041 (CN); JI, Xun, Chengdu, Sichuan 610041 (CN); HUANG, Wang, Chengdu, Sichuan 610041 (CN); LI, Wen-Bin, Chengdu, Sichuan (CN)
(74) Representative: Altmann Stößel Dick Patentanwälte PartG mbB
(86) International application number: PCT/CN2020/071345
(87) International publication number: WO 2020/143746

(56) References cited:
- CN-A- 101 703 497
- CN-A- 104 547 053
- CN-A- 107 033 001
- US-A1- 2019 255 007
- US-B2- 9 833 487
- DENIZ BAGDAS ET AL: "Antihyperalgesic activity of chlorogenic acid in experimental neuropathic pain", JOURNAL OF NATURAL MEDICINES, vol. 67, no. 4, 1 December 2012 (2012-12-01), pages 698-704, XP055717245, JP ISSN: 1340-3443, DOI: 10.1007/s11418-012-0726-z
- HARA KOJI ET AL: "Chlorogenic acid administered intrathecally alleviates mechanical and cold hyperalgesia in a rat neuropathic pain model", EUROPEAN JOURNAL OF PHARMACOLOGY, ELSEVIER SCIENCE, NL, vol. 723, 30 October 2013 (2013-10-30), pages 459-464, XP028607943, ISSN: 0014-2999, DOI: 10.1016/J.EJPHAR.2013.10.046
- ROSEVEARE DAVID: "World Congress on Osteoporosis, Osteoarthritis and Musculoskeletal Diseases (WCO-IOF-ESCEO 2018): Poster Abstracts", OSTEOPOROSIS INTERNATIONAL, SPRINGER LONDON, LONDON, vol. 29, no. Suppl 1, 1 April 2018 (2018-04-01), pages 149-565, XP037136683, ISSN: 0937-941X, DOI: 10.1007/S00198-018-4465-1 [retrieved on 2018-07-27]
- TAJIK NARGES ET AL: "The potential effects of chlorogenic acid, the main phenolic components in coffee, on health: a comprehensive review of the literature", EUROPEAN JOURNAL OF NUTRITION, STEINKOPFF VERLAG, DARMSTADT, DE, vol. 56, no. 7, 8 April 2017 (2017-04-08), pages 2215-2244, XP036321099, ISSN: 1436-6207, DOI: 10.1007/S00394-017-1379-1 [retrieved on 2017-04-08]
- PRASHANT SINGH CHAUHAN ET AL: "Differential Effects of Chlorogenic Acid on Various Immunological Parameters Relevant to Rheumatoid Arthritis", PHYSIOTHERAPY RESEARCH, vol. 26, no. 8, 19 December 2011 (2011-12-19), pages 1156-1165, XP055353569, GB ISSN: 0951-418X, DOI: 10.1002/ptr.3684
- DOS SANTOS MICHEL DAVID ET AL: "Evaluation of the anti-inflammatory, analgesic and antipyretic activities of the natural polyphenol chlorogenic acid", BIOLOGICAL AND PHARMACEUTICAL BULLETIN, vol. 29, no. 11, 29 November 2006 (2006-11-29), pages 2236-2240, XP055979971,
- LIU FEI ET AL: "Effects of chlorogenic acid on voltage-gated potassium channels of trigeminal ganglion neurons in an inflammatory environment", BRAIN RESEARCH BULLETIN, ELSEVIER SCIENCE LTD, OXFORD, GB, vol. 127, 9 September 2016 (2016-09-09), pages 119-125, XP029810186, ISSN: 0361-9230, DOI: 10.1016/J.BRAINRESBULL.2016.09.005
- TOTSCH STACIE K ET AL: "Immune system involvement in specific pain conditions", MOLECULAR PAIN, vol. 13, 1 January 2017 (2017-01-01), pages 1-17, XP055981398, GB ISSN: 1744-8069, DOI: 10.1177/1744806917724559 Retrieved from the Internet: URL:https://www.ncbi.nlm.nih.gov/pmc/artic les/PMC5555497/pdf/10.1177_174480691772455 9.pdf>
- ZHOU, Yimei et al: "Effect of Chlorogenic Acid on Peripheral Neuropathic Pain Induced by Paclitaxel", Journal of Hubei University of Science and Technology (Medical Sciences), vol. 33, no. 3, 15 June 2019 (2019-06-15), pages 188-191,277, XP055830324, ISSN: 2095--464
- Deniz Bagdas , Nilufer Cinkilic , Hasret Yucel Ozboluk , Musa Ozgur Ozyigit , Mine Sibel Gurun: "Antihyperalgesic Activity of Chlorogenic Acid in Experimental Neuropathic Pain", Journal of Natural Medicines, vol. 67, no. 4, 1 December 2012 (2012-12-01), pages 698-704, XP055717245, ISSN: 1861--029, DOI: 10.1007/s11418-012-0726-z

## Description

### Technical field

The present invention relates to chlorogenic acid for use for preventing or treating cancer pain, as well as pharmaceutical preparations and compositions comprising chlorogenic acid as an active ingredient for use in the prevention or treatment of cancer pain.

### Background art

Pain is a complex, common clinical symptom and disease that reflects subjective emotional feeling. In 1979, the International Association for the Study of Pain (IASP) defined pain as an unpleasant subjective feeling and emotional experience related to tissue damage or potential tissue damage. In 2016, the definition of pain was updated as: pain is a painful experience caused by actual or potential tissue damage, with sensory, emotional, cognitive, and social levels. In 1985, the American Pain Association proposed that pain is the fifth vital sign following heart rate, blood pressure, pulse, and breathing.

The classification of pain is more complicated. In 1994, the pain was classified by IASP according to the specific characteristics of pain, such as location, duration, sustained intensity, cause of the pain, and the pain caused by system disorders. Clifford Woolf believes that the systematic classification of IASP is not enough to guide research and treatment, and divides pain into a nociceptive pain, a neuropathic pain, and an inflammatory pain. If classified according to duration, pain is divided into an acute pain and a chronic pain, and generally refers to chronic pain in clinical. In the new ICD classification method, chronic pain is divided into the following 7 categories: 1) chronic primary pain; 2) chronic cancer pain; 3) chronic postoperative pain and post-traumatic pain; 4) neuropathic pain; 5) chronic head and maxillofacial pain; 6) chronic visceral pain; 7) chronic skeletal muscle pain.

Among them, cancer pain is a syndrome caused by a series of different pathophysiological changes, including early inflammatory pain, as well as neuropathic pain caused by sensory and sympathetic nerve damage, visceral pain, bone injury, cell death, bone destruction and bone pain as the course of the disease progresses, and at the same time causing anxiety, fear, cognitive impairment, etc., due to pain and emotional changes. Regarding the treatment of cancer pain, American NCCN "Clinical Guidelines in Oncology" (2018 Edition) emphasizes that pain management should reach 5A goal, namely, optimize analgesia (Analgesia), optimize activities of daily living (Activities), minimize adverse effects (Adverse effects), avoid aberrant drug taking (Aberrant drug taking), and relationship between pain and mood (Affect).

In 2011, the Ministry of Health of China formulated and issued "Cancer Pain Diagnosis and Treatment Standards" (2011 edition), pointing out that cancer pain has various causes, which can be roughly divided into three categories: 1) Tumor-related pain: caused by tumor directly invading and oppressing local tissues, tumor metastasis affecting bone tissue, and the like. 2) Pain associated with tumor treatment: commonly caused by surgery, traumatic examinations, radiotherapy, and treatment with cytotoxic chemotherapy drugs. 3) Non-tumor-related pain: including the pain caused by other comorbidities, complications, and other non-tumor factors.

Cancer pain is a complex pathological process. It is currently speculated that cancer pain is caused by the production and secretion of pain mediators by cancer cells in the tumor microenvironment. At the same time, the chemokines or mediators released by tumor cells can recruit other cells such as nerve cells, lymphocytes, endothelial cells, and fibroblasts, and further secrete mediators, such as tumor necrosis factor α (TNF-α), prostaglandin E (PGE), endothelin (ET), interleukin 1 (IL-1), interleukin 6 (IL-6), epithelial growth factor, transforming growth factor B, platelet-derived growth factor, adenosine triphosphate (ATP), nerve growth factor (NGF), etc. These mediators sensitize or activate specific receptors on primary afferent sensory neurons, and plays a role in the body, leading to the occurrence and maintenance of cancer pain.

Therefore, cancer pain assessment is a reasonable and effective analgesic treatment prerequisite, and its assessment should follow the assessment principles of "routine, quantitative, comprehensive and dynamic". The treatment of cancer pain should also adopt the principle of comprehensive treatment. According to the patient's conditions and physical state, analgesic treatment means can be used, to continuously and effectively eliminate pain, prevent and control adverse drug reactions, reduce the psychological burden caused by pain and treatment, and improve the life quality of patients to the maximum extent possible.

Chronic skeletal muscle pain refers to persistent or recurrent pain caused by bone, joint, muscle or other related soft tissue diseases, which belongs to nociceptive pain. Bone joint pain includes and is not limited to knee joint pain, ankle joint pain, wrist joint pain, elbow joint pain, shoulder joint pain, patellar joint pain, hip joint pain, femoral joint pain, cervical spine, and lumbar spine pain, etc. There are many causes, such as compressive neuropathic pain or somatic referred pain. The pain included in this part has the following characteristics: containing persistent inflammation caused by infectious, autoimmune, or metabolic causes, such as rheumatoid arthritis and gouty inflammation; as well as structural changes that affect bones, joints, tendons or muscles, such as osteoarthritis, soft tissue injury, strain and so on.

At present, the therapeutic methods of cancer pain include an etiological treatment, a drug analgesic therapy and a non-drug therapy (2011 edition of cancer pain diagnosis and treatment standards). Cancer pain is a kind of complication caused by a cancer itself, an anticancer treatment for cancer patients, such as surgery, radiotherapy, or chemotherapy, may remove or relieve the cancer pain. Drug analgesic therapy is a three-step analgesic treatment plan formulated by WHO according to cancer pain. According to the pain degree of patients, the analgesic drugs with different intensity were selected. For mild pain, non-steroidal anti-inflammatory drugs (NSAIDs) can be used; for moderate pain, weak opioids can be selected, that may be used in combination with NSAIDs; for severe pain, strong opioids can be selected, that may be used in combination with NSAIDs. NSAIDs are essential drugs for the treatment of mild cancer pain, including ibuprofen, diclofenac, indomethacin, celecoxib, and paracetamol, whose common adverse reactions are a peptic ulcer, a gastrointestinal bleeding, a platelet dysfunction, a kidney function damage, a liver function damage, etc. Opioids are the chosen drug for the treatment of moderate and severe pain. At present, the short-acting drugs used clinically for moderate to severe cancer pain are immediate-release morphine tablets, and the long-acting drugs are morphine sustained-release tablets, oxycodone sustained-release tablets, fentanyl transdermal patches and the like, whose adverse reactions mainly include constipation, nausea, vomiting, lethargy, itching, urinary retention, delirium, cognitive impairment, respiratory depression, etc.

Cancer pain is mostly a chronic pain. Although WHO three-step analgesic therapy is used clinically to treat cancer pain, the pain control effect is not very satisfactory. 36-50% of cancer patients still endure various degrees of pain. Moreover, three-step analgesics (opioids and NSAIDs) also have serious adverse reactions. Due to the mental dependence of opioid analgesics, that is, addiction, countries around the world have also introduced corresponding drug management programs to strengthen the management and use of opioids.

The drugs used to treat bone and joint pain are similar to those used to treat a cancer pain. Both are acetaminophen, COX-2 inhibitors, NSAIDs and opioids. The treatment strategy is still WHO's three-step analgesic therapy. Obviously, while receiving treatment, patients will also suffer from the toxic side effects of these drugs. Therefore, it is extremely urgent to develop a safe and effective drug for the treatment of pain.

Tajik, Narges et al. review the wide range of potential health benefits of chlorogenic acid, including its anti-diabetic, anti-carcinogenic, anti-inflammatory and anti-obesity impacts and postulate that said agent may provide a non-pharmacological and non-invasive approach for treatment or prevention of some chronic diseases (Tajik, Narges, et al. "The potential effects of chlorogenic acid, the main phenolic components in coffee, on health: a comprehensive review of the literature." European journal of nutrition 56 (2017): 2215-2244).

### Content of the invention

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods for treatment of the human body by therapy, according to the present invention.

In order to solve the above problem, the present invention first provides the use of chlorogenic acid in preparing drugs or pharmaceutical compositions for preventing or treating cancer pain.

Further, said cancer pain is a neuropathic pain.

Further, said cancer pain is a nociceptive pain.

Further, said cancer pain is a chronic pain.

As the use mentioned above, preferably, the prevention or the treatment of pain is to reduce mechanical hyperalgesia or radiant hyperalgesia caused by cancer.

As the use mentioned above, the drug or pharmaceutical composition is those inhibiting the expression of tumor necrosis factor α (alpha) (TNF-α), interleukin 1β (beta) (IL-1β), and interleukin 6 (IL-6).

As the use mentioned above, the drug or pharmaceutical composition is those regulating/increasing the expression of 5-hydroxytryptamine (5-HT) and dopamine (DA) caused by pain.

The present invention also provides a drug for the prevention or the treatment of cancer pain, and the drug is a pharmaceutical preparation obtained by using chlorogenic acid as an active ingredient, with addition of pharmaceutically acceptable excipients.

Preferably, the prevention or treatment of pain is to reduce mechanical hyperalgesia or radiant hyperalgesia caused by cancer.

For the drug mentioned above, in the pharmaceutical preparation, each preparation unit contains 1 to 3000 mg of chlorogenic acid; and the preparation unit is a pill, a tablet, a packet, a small ball, an ampule or a bottle.

For the drug mentioned above, the human dosage of chlorogenic acid is 1 to 10 mg/kg in the pharmaceutical preparation.

For the drug mentioned above, the pharmaceutical preparation is an oral preparation or an injection.

The present invention also provides a pharmaceutical composition for preventing or treating cancer pain, characterized in that it is a pharmaceutical composition containing the pharmaceutical preparation mentioned above.

The inventor surprisingly discovered that chlorogenic acid can effectively prevent and treat pain, especially cancer pain as well as bone and joint pain. Therefore, the use of chlorogenic acid provided by the present invention in the preparation of drugs for preventing or treating cancer pain has good industrialization value.

In the case of a continuous medication, the drug of the present invention can effectively relieve cancer pain without toxic side effects. It can replace analgesic drugs such as opioids and non-steroidal anti-inflammatory drugs, avoid the toxic and side effects produced by long-term use of opioids, improve the life quality of patients, and have good clinical application prospects.

By the following specific examples of said embodiments, the above content of the present invention is further illustrated. But it should not be construed that the scope of above subject of the present invention is limited to the following examples. The techniques realized based on the above content of the present invention are all within the scope of the present invention.

### Description of Figures

Figure 1. The curve diagram of the paw withdrawal threshold of rats due to mechanical hyperalgesia in each experimental group of Experimental Example 3.
Figure 2. The curve diagram of radiant heat pain threshold of rats in each experimental group of Experimental Example 3.
Figure 3. Histogram of TNF-α expression in serum of rats in each experimental group of Experimental Example 3.
Figure 4. Histogram of IL-1β expression in serum of rats in each experimental group of Experimental Example 3.
Figure 5. Histogram of IL-6 expression in serum of rats in each experimental group of Experimental Example 3.
Figure 6. Histogram of NE content in brain tissue of rats in each experimental group of Experimental Example 3.
Figure 7. Histogram of DA content in brain tissue of rats in each experimental group of Experimental Example 3.
Figure 8. Histogram of 5-HT content in brain tissue of rats in each experimental group of Experimental Example 3.

### Examples

The starting materials and equipment used in the specific examples of the present invention are all known products and can be obtained by purchasing commercially available products.

### Example 1 The formula for oral preparation of the present invention

### 1. Formula 1

Chlorogenic acid 1000 g.

Preparative method: chlorogenic acid was aseptically weighed and subpacked as powders.

### 2. Formula 2

Chlorogenic acid 1000 g, bulking agent 500 g, binding agent 5 g.

Preparative method: chlorogenic acid, bulking agent, and binding agent were weighed according to the formula, granulated, sieved, and subpacked as granules.

### 3. Formula 3

Chlorogenic acid 1000 g, bulking agent 500 g, binding agent 5 g, and lubricant 3 g.

Preparative method: chlorogenic acid, bulking agent, and binding agent were weighed according to the formula, granulated, sieved, and then lubricant was added, followed by pressing, to obtain tablets.

Above bulking agents were one or more of mannitol, lactose, starch, microcrystalline cellulose, and dextrin; the binding agents were sodium carboxymethylcellulose and PVP; the lubricants were magnesium stearate, talcum powder, and micro silica gel.

### Example 2 The formula for injection of the present invention

### 1. Formula 1

Chlorogenic acid 1000 g.

Preparative method (1): chlorogenic acid was aseptically weighed according to the formula, and aseptically subpacked as a powder injection.

Preparative method (2): chlorogenic acid was weighed according to the formula, dissolved in water for injection, filtered, sterilized, freeze-dried, and filled, to obtain freeze-dried powder injection.

### 2. Formula 2

Chlorogenic acid 1000 g, stent agent 2667 g, and antioxidant 67 g.

Preparative method: chlorogenic acid, stent agent, and antioxidant were weighed according to the formula, dissolved in water for injection, filtered, sterilized, filled, and freeze-dried to obtain freeze-dried powder injection.

Said stent agents were mannitol, lactose and glucose; the antioxidants were sodium bisulfite, vitamin, glutathione, and folic acid.

### Example 3 Animal experiment of chlorogenic acid on preventing or treating bone cancer pain in rats

### 1. Experimental material

### 1.1 Animals

SD rats, female, weighing 180-200 g, purchased from Chengdu Dossy Experimental Animal Co., Ltd.

### 1.2 Cell lines

Walker rat breast cancer cell lines, purchased from Shanghai Institutes for Biological Sciences, Chinese Academy of Sciences.

### 1.3 Drugs

Chlorogenic acid, batch No.: 171101, with a content of 99.83%, prepared by Sichuan Jiuzhang Biological Science and Technology Co., LTD.

### 2. Experimental method

### 2.1 Preparation of cell suspension

After two SD female rats were intraperitoneally injected with 0.5 mL cancer cells (4×104 cells/µL), ascites was collected on the 7^{th} day, and the cells were rinsed 3 times with sterile 0.01 mol/L PBS to adjust to the concentration of 4×103 cells/µL for use.

### 2.2 Model building

60 SD rats were randomly selected, and after anesthesia, a small 1 cm incision was made in the upper tibial skin. A 10 mL syringe needle was used to puncture and perforate, then a 10 µL syringe was inserted into the bone marrow cavity, and 3 µL cell suspension containing 3 × 103 Walker rat breast cancer cells was slowly injected. The pinhole was sealed with bone wax, and the skin was sutured. After surgery, the rats were placed on a 37 °C hot plate for rewarming. After recovery, rats were returned to the cage for further breeding, to obtain the model group. In addition, 8 SD rats were randomly selected, and an equal volume of PBS solution was injected into the marrow cavity of the left upper tibia. The rest of the operations were the same as the model group, to obtain the sham operation group.

From the 10^{th} day after the operation, the rats in the model group with mechanical hyperalgesia and radiant thermal pain were screened out by using pain behavior as the criterion.

Mechanical pain hypersensitivity: a continuous pain measurement was performed from the 10^{th} day after the operation, the contact stimulator's fine fibers were allowed to touch with the middle of the affected side of the rat's plantar, and the force can be increased up to 80 g within 5 seconds. The rat's paw withdrawal respond was observed, and the paw withdrawal threshold was recorded. Each animal was tested 5 times, with an interval of 5 min between two tests. The paw withdrawal threshold of the model group was significantly different from that of the sham operation group, indicating that there was mechanical hyperalgesia.

Radiation thermal pain response: the rat was placed on a glass plate, and the affected side of the foot was irradiated with a heat radiator. The plantar side of each rat would be tested 3 times at an interval of 5 min. The response time from exposure to paw withdrawal was recorded, and the time to retract the paw was the pain threshold. The pain threshold of the model group was significantly different compared with that of the sham operation group, indicating that there was a radiation thermal pain response.

### 2.3 Administration of animals

The rats that had been successfully modeled were randomly divided into groups, eight rats for each group, that included high-dose chlorogenic acid group (80 mg/kg), middle-dose chlorogenic acid group (40 mg/kg), low-dose chlorogenic acid group (20 mg/kg), model negative group (N.S normal saline), and sham operation group (N.S normal saline). The rats were intraperitoneally injected with drugs for 15 days in a volume of 0.2 mL/10 g, once a day; the model-negative group and the sham operation group were given the same volume of normal saline.

### 2.4 Detection of indexes

### 2.4.1 Pain behavior

From the second day after administration, the mechanical pain hypersensitivity and radiation thermal pain were investigated before each administration, and the paw withdrawal threshold and the pain threshold were recorded.

### 2.4.2 Cytokines

After the last administration, the blood was collected from the eyeballs of the rats, and the animals in each group were sacrificed by neck removal. The blood samples were allowed to stand at room temperature for 20 minutes and then centrifuged. The supernatant was collected and the contents of TNF-α, IL-1β, and IL-6 in the peripheral blood serum of the mice were determined by ELISA.

After the rats were sacrificed, the hypothalamus was quickly separated, homogenized in an ice bath with perchloric acid, and centrifuged. The supernatant was collected, and the contents of NE, DA, and 5-HT were determined by HPLC-EC method (Hou Yanning, Wang Na, et al, Effects of progesterone on morphine-induced conditioned place preference and levels of monoamine transmitters in a rat brain. Chinese Journal of Pharmacology, 2006, 22(8), 980-983).

### 3. Statistical processing

All data were expressed as
x̅ ± s. The SPSS 11.0 software was used for the statistical processing and the t test analysis. P <0.05 was considered to be significantly different.

### 4. Experimental results

### 4.1 Pain behavior

### 4.1.1 Mechanical pain hypersensitivity

In the model group selected after operation, during the administration period, the mechanical paw withdrawal threshold was significantly different compared with the sham operation group (p <0.05), indicating that the tibia implant model was successful.

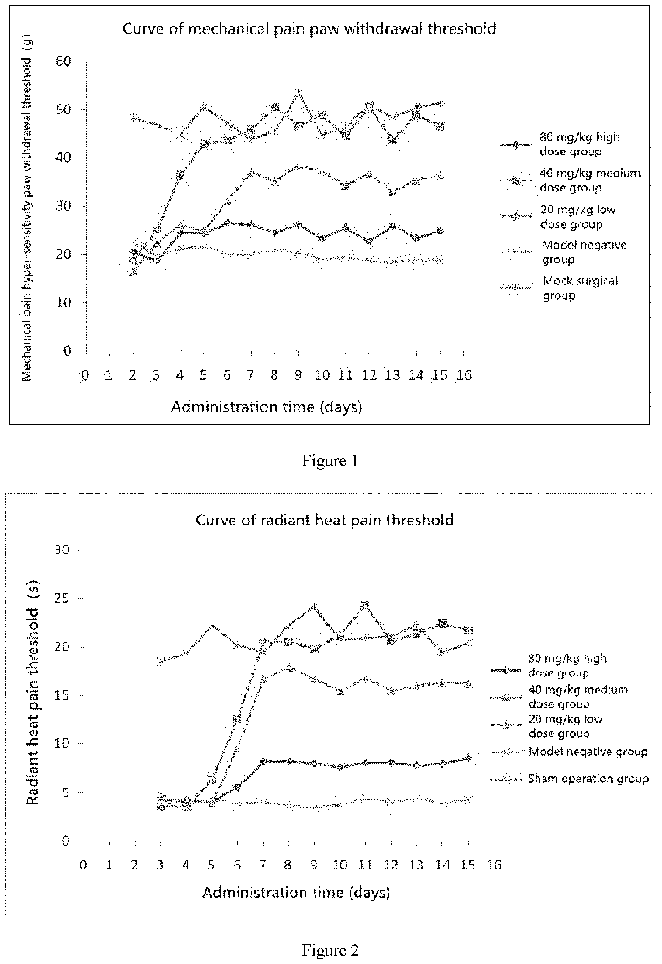

The 40 mg/kg and 20 mg/kg dose groups of chlorogenic acid had a significant difference in paw withdrawal thresholds compared with the model group, from the 5^{th} to 7^{th} days after administration (p <0.05), and especially 40 mg/kg. The dose group was basically the same as the sham operation group; it showed that the administration dose (20 to 40 mg/kg) of chlorogenic acid could significantly improve and alleviate the mechanical hyperalgesia caused by bone cancer, that was in a dose-effect relationship. In the 80 mg/kg dose group, the mechanical paw withdrawal threshold was different from the model group, but the difference was not statistically significant (p > 0.05). The results were shown in Table 1 and Figure 1.

**Table 1. The paw withdrawal threshold of mechanical hyperalgesia in rats of each experimental group (x̅ ± s).**

| | Dose | Paw withdrawal threshold (g) | | | | | | | | | | | | | |
|---|---|---|---|---|---|---|---|---|---|---|---|---|---|---|---|
| Groups | (m g/kg ) | 2d | 3d | 4d | 5d | 6d | 7d | 8d | 9d | 10d | 11d | 12d | 13d | 14d | 15d |
| Chlorogenic acid high dose group | 80 | 20.5 8 ± 4.73 | 18.59 ± 5.14 | 24.41 ± 5.26 | 24.39 ± 4.15 | 26.53 ± 5.19 | 26.07 ± 3.15 | 24.52 ± 7.01 | 26.11 ± 4.88 | 23.21 ± 3.45 | 25.42 ± 3.74 | 22.63 ± 5.23 | 25.79 ± 3.42 | 23.25 ± 2.45 | 24.8 3 ± 3.89 |
| Chlorogenic acid middle dose group | 40 | 18.5 3 ± 3.04 | 24.94 ± 4.43 | 36.37 ± 2.71 | 42.83 ± 5.14 | 4.3.59 ± 3.83 | 45.85 ± 7.25 | 50.41 ± 6.79 | 46.49 ± 2.86 | 48.77 ± 6.62 | 44.59 ± 7.11 | 50.52 ± 4.04 | 43.72 ± 7.16 | 48.73 ± 6.51 | 46.5 1 ± 3.38 |
| Chlorogenic acid low dose group | 20 | 16.4 6 ± 3.73 | 22.32 ± 4.15 | 26.14 ± 4.11 | 24.86 ± 3.51 | 31.21 ± 5.82 | 37.06 ± 3.45 | 35.11 ± 2.21 | 38.48 ± 3.76 | 37.23 ± 6.82 | 34.22 ± 5.38 | 36.74 ± 4.28 | 33.02 ± 4.41 | 35.43 ± 2.42 | 36.5 2 ± 4.05 |
| Model negative group | N.S | 22.4 ± 2.36 | 19.85 ± 4.23 | 21.08 ± 7.11 | 21.63 ± 3.06 | 20.12 ± 4.29 | 1988 ± 5.26 | 21.04 2 ± 6 2.59 | 20.46 ± 3.08 | 1889 ± 2.95 | 1927 ± 6.08 | 18.75 ± 3.47 | 18.29 ± 536 | 18.82 ± 3.11 | 18.6 ± 3.51 |
| Sham operation group | N.S | 48.2 3 ± 7.79 | 46.79 ± 5.56 | 44.83 ± 9.2; | 50.52 ± 8.38 | 47.04 ± 3.23 | 43.84 ± 6.91 | 45.52 ± 4.08 | 53.41 ± 6.25 | 44.75 ± 8.44 | 46.42 ± 6.09 | 51.03 ± 3.14 | 48.39 ± 4.25 | 50.52 ± 4.08 | 51.2 3 ± 5.29 |

| | | | | | | | | | | | | | | | |
|---|---|---|---|---|---|---|---|---|---|---|---|---|---|---|---|
| Note: N.S, no drug. | | | | | | | | | | | | | | | |

### 4.1.2 Radiant heat pain response

The 40 mg/kg and 20 mg/kg dose groups of chlorogenic acid had a significant difference in radiant heat pain thresholds compared with the model group from 7 days after administration (p <0.05), and 40 mg/kg dose group was the most significant, and basically equivalent to the sham operation group. It showed that the administration dose (20 to 40 mg/kg) of chlorogenic acid could significantly improve and alleviate the radiant heat hyperalgesia caused by bone cancer, that was in a dose-effect relationship. In the 80 mg/kg dose group, the mechanical paw withdrawal threshold was different from the model group, but the difference was not statistically significant (p > 0.05). The results were shown in Table 2 and Figure 2.

**Table 2. The pain threshold of radiant heat pain reaction in rats of each experimental group (x̅ ± s).**

| Groups | Dose | Pain threshold (s) | | | | | | | | | | | | | |
|---|---|---|---|---|---|---|---|---|---|---|---|---|---|---|---|
| | (mg /kg) | 2 d | 3 d | 4 d | 5 d | 6 d | 7 d | 8 d | 9 d | 10 d | 11 d | 12 d | 13 d | 14 d | 15 d |
| Chlorogenic acid high dose group | 80 | 4.17 ± 2.21 | 4.24 ± 2.21 | 4.09 ± 1.96 | 5.52 ± 3.05 | 8.15 ± 2.58 | 8.22 ± 3.05 | 7.98 ± 2.52 | 7.59 ± 3.19 | 8.03 ± 3.22 | 8.05 ± 1.11 | 7.76 ± 2.52 | 7.98 ± 3.41 | 8.52 ± 2.12 | 8.38 ± 3.74 |
| Chlorogenic acid middle dose group | 40 | 3.62 ± 2.43 | 3.52 ± 2.42 | 6.36 ± 3.41 | 12.52 ± 4.01 | 20.53 ± 7.41 | 20.49 ± 5.72 | 19.83 ± 6.09 | 21.21 ± 3.64 | 24.32 ± 8.41 | 20.59 ± 6.77 | 21.41 ± 3.62 | 22.39 ± 6.11 | 21.74 ± 3.98 | 20.88 ± 6.43 |
| Chlorogenic acid low dose group | 20 | 3.86 ± 2.28 | 4.11 ± 3.26 | 3.93 ± 4.55 | 9.53 ± 5.62 | 16.66 ± 4.38 | 17.89 ± 3.52 | 16.72 ± 4.09 | 15.43 ± 2.52 | 16.74 ± 5.21 | 15.52 ± 2.73 | 15.97 ± 3.08 | 16.35 ± 4.54 | 16.22 ± 2.25 | 16.75 ± 1.81 |
| Model negative group | N.S | 4.74 ± 1.28 | 3.79 ± 2.00 | 4.21 ± 2.05 | 3.88 ± 2.51 | 4.02 ± 1.63 | 3.62 ± 1.56 | 3.43 ± 3.13 | 3.76 ± 2.93 | 4.42 ± 2.26 | 4.05 ± 1.88 | 4.41 ± 2.92 | 3.98 ± 2.06 | 4.22 ± 2.19 | 4.38 ± 2.77 |
| Sham operation group | N.S | 18.48 ± 2.23 | 19.29 ± 3.71 | 22.21 ± 3.04 | 20.2 ± 3.11 | 19.45 ± 4.83 | 22.27 ± 2.58 | 24.16 ± 5.52 | 20.68 ± 3.85 | 20.93 ± 4.79 | 21.12 ± 3.52 | 22.29 ± 5.83 | 19.41 ± 2.63 | 20.43 ± 2.33 | 22.62 ± 3.52 |

| | | | | | | | | | | | | | | | |
|---|---|---|---|---|---|---|---|---|---|---|---|---|---|---|---|
| Note: N.S, no drug. | | | | | | | | | | | | | | | |

### 4.2 Cytokines

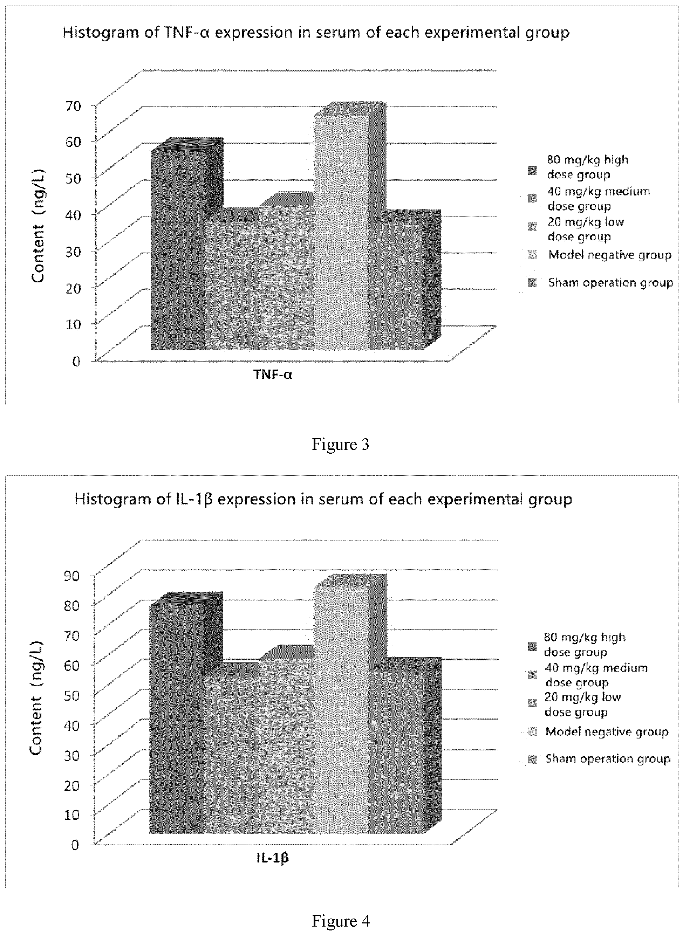

After 15 days of administration in each test group, the serum levels of TNF-α, IL-1β and IL-6 in the 40 mg/kg and 20 mg/kg dose groups of chlorogenic acid were lower than those in the model-negative group, and the difference was significant (p < 0.05), but for 80 mg/kg dose group, the difference was not significant. The results were shown in Table 3, Figure 3, Figure 4, and Figure 5.

**Table 3. The cytokine content in serum of rats in each experimental group**

| Groups | Dose (mg/kg) | Cytokines (ng/L) | | |
|---|---|---|---|---|
| | | TNF-α | 1L-1β | IL-6 |
| Chlorogenic acid high dose | 80 group | 54.48 ± 5,994 | 76.25 ± 4.62# | 98.82 + 4.54# |
| Chlorogenic acid middle dose group | 40 | 35.25 ± 4.62^{∗} | 52.71 ± 6.39^{∗} | 78.73 ± 6.39^{∗} |
| Chlorogenic acid low dose group | 20 | 39.74 ± 6.04^{∗} | 58.54 ± 8.23^{∗} | 84.09 ± 5.16^{∗} |
| Model negative group | - | 64.32 ± 2.47# | 82.37 ± 5,889 | 112.56 ± 6.55# |
| Sham operation group | - | 34.86 ± 4.39 | 54.43 ± 4.58 | 78.81 ± 8.32 |

| | | | | |
|---|---|---|---|---|
| Note: * means p < 0.05 compared with the model negative group, # means p < 0.05 compared with the sham operation group. | | | | |

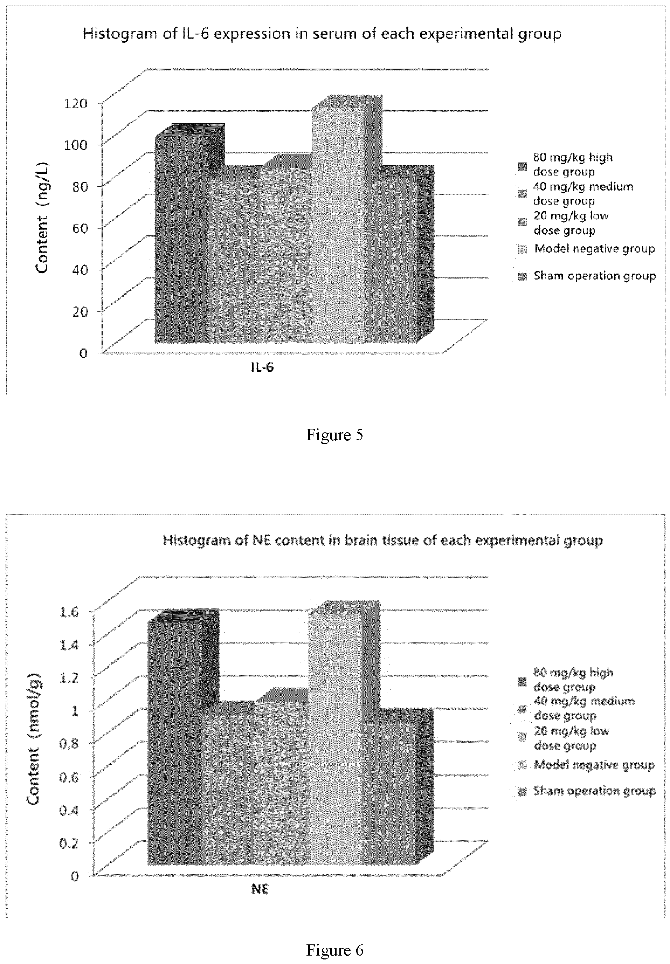

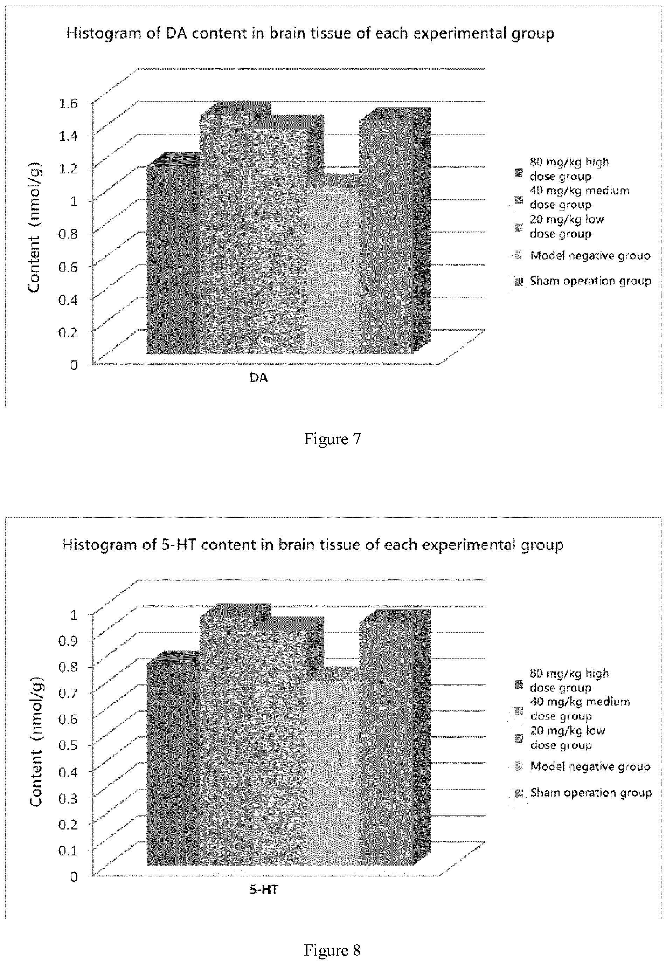

The contents of NE, DA, and 5-HT in the brain tissues of rats in the 40 mg/kg and 20 mg/kg dose groups of chlorogenic acid were basically the same as those in the sham operation group, without significant difference (p > 0.05), but compared with the model-negative group, the difference was significant (p < 0.05). There was no significant difference between the 80 mg/kg dose group and the model-negative group (p > 0.05), while the 80 mg/kg dose group had a significant difference from the sham operation group (p <0.05). The results were shown in Table 4, Figure 6, Figure 7, and Figure 8.

**Table 4. The contents of NE, DA and 5-HT in the brain tissue of rats in each test group (x̅± s)**

| Groups | Dose (mg/kg) | Cytokines (nmol/g) | | |
|---|---|---|---|---|
| | | NE | DA | 5-HT |
| Chlorogenic acid high dose group | 80 | 1.47 ± 0.26# | 1.15 ± 0.12# | 0.77 ± 0.11# |
| Chlorogenic acid middle dose group | 40 | 0.91 ± 0.15^{∗} | 1.46 ± 0.08^{∗} | 0.95 ± 0.19^{∗} |
| Chlorogenic acid low dose group | 20 | 0.99 + 0.18^{∗} | 1.38 ± 0.21^{∗} | 0.91 ± 0.15^{∗} |
| Model negative group | - | 1.52 ± 0.21# | 1.02 ± 0.26# | 0.71 ± 0.20# |
| Sham operation group | - | 0.86 ± 0.23 | 1.43 ± 0.14 | 0.93 ± 0.09 |

| | | | | |
|---|---|---|---|---|
| Note: * means p < 0.05 compared with the model negative group, # means p < 0.05 compared with the sham operation group. | | | | |

### 5. Summary

In each test group of the tibial implant model, among the indicators of mechanical hyperalgesia and radiant heat pain, the 20 to 40 mg/kg dose groups of chlorogenic acid had significant differences compared with the model-negative group, in which the 40 mg/kg dose group was equivalent with the sham operation group, indicating that chlorogenic acid could effectively improve and relieve a cancer pain, and presented a dose-effect relationship within a certain dose range.

In the process of cancer pain, cytokines TNF-α, IL-1β, and IL-6 played important regulatory roles and were important regulatory mediators in the neuro-endocrine-immune function system. Noxious external stimuli would increase their expression, and their contents were directly related to the degree of cancer pain.

In each test group, the expression of TNF-α, IL-1β, and IL-6 detected in the serum of rats in the 20-40 mg/kg of chlorogenic acid dose groups was significantly different from that of the model-negative group, and did not show significant difference from the sham operation group, indicating that chlorogenic acid could effectively regulate the serum levels of TNF-α, IL-1β, and IL-6, and maintain their normal levels. Thus, the chlorogenic acid was expected to be able to improve and relieve the cancer pain, that was consistent with the experimental results of the pain behavior.

In the brain tissue, NE, DA, and 5-HT were important participants in the cellular biological pathways related to pain perception. The contents of NE, DA, and 5-HT in the brain tissue of the 20-40 mg/kg of chlorogenic acid dose groups were basically same as those in the sham operation group, and there was no significant difference. However, compared with the negative model group, their contents were significant differences, indicating chlorogenic acid could induce the expression of DA and 5-HT caused by pain, and reduce the expression of NE. Thus, the chlorogenic acid was shown to have the ability of improving and relieving pain, which was consistent with the experimental results of pain behavior.

In summary, the chlorogenic acid could effectively ameliorate and alleviate pain, and could be used to prepare the drug or pharmaceutical composition of the present invention for preventing or treating pain. The drug of the present invention could effectively reduce mechanical hyperalgesia and radiant hyperalgesia caused by cancer, and had no toxic side effects. It could replace analgesic drugs such as opioids and non-steroidal anti-inflammatory drugs, and avoid the toxic and addictive effects due to long-term use of opioids, improve the life quality of patients, and have good clinical application prospects.

## Claims

1. Chlorogenic acid for use for preventing or treating cancer pain.

2. Chlorogenic acid for use according to claim 1, **characterized in that** said cancer pain is a neuropathic pain, a nociceptive pain or a chronic pain.

3. Chlorogenic acid for use according to claim 1, **characterized in that** the prevention or the treatment of pain is to reduce mechanical hyperalgesia or radiant hyperalgesia caused by cancer.

4. Chlorogenic acid for use according to claim 1, **characterized in that** the chlorogenic acid is used as a drug or pharmaceutical composition for inhibiting the expression of tumor necrosis factor α, interleukin 1β, and interleukin 6.

5. Chlorogenic acid for use according to claim 1, **characterized in that** the chlorogenic acid is used as a drug or pharmaceutical composition for regulating/increasing the expression of 5-hydroxytryptamine and dopamine caused by pain;
and/or as a drug or pharmaceutical composition for regulating/reducing the expression of noradrenaline caused by pain.

6. A pharmaceutical preparation for use as a drug for the prevention or the treatment of cancer pain, **characterized in that** it is obtained by using chlorogenic acid as an active ingredient, with addition of pharmaceutically acceptable excipients;
preferably the prevention or the treatment of pain being to reduce mechanical hyperalgesia or radiant hyperalgesia caused by cancer.

7. The pharmaceutical preparation for use according to claim 6, **characterized in that** in the preparation, each preparation unit contains 1 to 3000 mg of chlorogenic acid; and the preparation unit is a pill, a tablet, a packet, a small ball, an ampule or a bottle.

8. The pharmaceutical preparation for use according to claim 7, **characterized in that** in the preparation, the human dosage of chlorogenic acid is 1 to 10 mg/kg.

9. The pharmaceutical preparation for use according to claim 7, **characterized in that** the preparation is an oral preparation or an injection.

10. A pharmaceutical composition for use as a drug for preventing or treating cancer pain, **characterized in that** it contains the preparation according to any one of claims 6 to 9.

## Patentansprüche

1. Chlorogensäure zur Verwendung bei der Prävention oder Behandlung von Krebsschmerzen.

2. Chlorogensäure zur Verwendung gemäß Anspruch 1, **dadurch gekennzeichnet, dass** es sich bei den Krebsschmerzen um neuropathische Schmerzen, nozizeptive Schmerzen oder chronische Schmerzen handelt.

3. Chlorogensäure zur Verwendung nach Anspruch 1, **dadurch gekennzeichnet, dass** die Prävention oder die Behandlung von Schmerzen darin besteht, die durch Krebs verursachte mechanische Hyperalgesie oder Strahlungshyperalgesie zu verringern.

4. Chlorogensäure zur Verwendung nach Anspruch 1, **dadurch gekennzeichnet, dass** die Chlorogensäure als Arzneimittel oder pharmazeutische Zusammensetzung zur Hemmung der Expression von Tumornekrosefaktor α, Interleukin 1β und Interleukin 6 verwendet wird.

5. Chlorogensäure zur Verwendung nach Anspruch 1, **dadurch gekennzeichnet, dass** die Chlorogensäure als Arzneimittel oder pharmazeutische Zusammensetzung zur Regulierung/Erhöhung der schmerzbedingten Expression von 5-Hydroxytryptamin und Dopamin
und/oder als Arzneimittel oder pharmazeutische Zusammensetzung zur Regulierung/Reduzierung der schmerzbedingten Expression von Noradrenalin verwendet wird.

6. Pharmazeutische Zubereitung zur Verwendung als Arzneimittel zur Prävention oder Behandlung von Krebsschmerzen, **dadurch gekennzeichnet, dass** sie durch Verwendung von Chlorogensäure als Wirkstoff unter Zusatz von pharmazeutisch unbedenklichen Exzipienten erhalten wird;
wobei die Prävention oder Behandlung von Schmerzen vorzugsweise darin besteht, die durch Krebs verursachte mechanische Hyperalgesie oder Strahlungshyperalgesie zu verringern.

7. Pharmazeutische Zubereitung zur Verwendung nach Anspruch 6, **dadurch gekennzeichnet, dass** in der Zubereitung jede Zubereitungseinheit 1 bis 3000 mg Chlorogensäure enthält und es sich bei der Zubereitungseinheit um eine Pille, eine Tablette, ein Päckchen, eine kleine Kugel, eine Ampulle oder eine Flasche handelt.

8. Pharmazeutische Zubereitung zur Verwendung nach Anspruch 7, **dadurch gekennzeichnet, dass** die Humandosis von Chlorogensäure in der Zubereitung 1 bis 10 mg/kg beträgt.

9. Pharmazeutische Zubereitung zur Verwendung nach Anspruch 7, **dadurch gekennzeichnet, dass** es sich bei der Zubereitung um eine orale Zubereitung oder eine Injektionszubereitung handelt.

10. Pharmazeutische Zusammensetzung zur Verwendung als Arzneimittel zur Prävention oder Behandlung von Krebsschmerzen, **dadurch gekennzeichnet, dass** sie die Zubereitung nach einem der Ansprüche 6 bis 9 enthält.

## Revendications

1. Acide chlorogénique pour utilisation pour la prévention ou le traitement d'une douleur cancéreuse.

2. Acide chlorogénique pour utilisation selon la revendication 1, **caractérisé en ce que** ladite douleur cancéreuse est une douleur neuropathique, une douleur nociceptive ou une douleur chronique.

3. Acide chlorogénique pour utilisation selon la revendication 1, **caractérisé en ce que** la prévention ou le traitement de la douleur est une réduction d'une hyperalgésie mécanique ou d'une hyperalgésie radiante causée par un cancer.

4. Acide chlorogénique pour utilisation selon la revendication 1, **caractérisé en ce que** l'acide chlorogénique est utilisé en tant que médicament ou composition pharmaceutique pour inhiber l'expression du facteur de nécrose tumorale α, de l'interleukine 1β, et de l'interleukine 6.

5. Acide chlorogénique pour utilisation selon la revendication 1, **caractérisé en ce que** l'acide chlorogénique est utilisé en tant que médicament ou composition pharmaceutique pour réguler/augmenter l'expression de la 5-hydroxytryptamine et de la dopamine causée par la douleur ;
et/ou en tant que médicament ou composition pharmaceutique pour réguler/réduire l'expression de la noradrénaline causée par la douleur.

6. Préparation pharmaceutique pour utilisation en tant que médicament pour la prévention ou le traitement d'une douleur cancéreuse, **caractérisée en ce qu'**elle est obtenue en utilisant un acide chlorogénique en tant que substance active, avec l'ajout d'excipients pharmaceutiquement acceptables ;
de préférence la prévention ou le traitement de la douleur étant destiné à réduire l'hyperalgésie mécanique ou l'hyperalgésie radiante causée par un cancer.

7. Préparation pharmaceutique pour utilisation selon la revendication 6, **caractérisée en ce que** dans la préparation, chaque unité de préparation contient 1 à 3000 mg d'acide chlorogénique ; et l'unité de préparation est une pilule, un comprimé, un paquet, une petite bille, une ampoule ou un flacon.

8. Préparation pharmaceutique pour utilisation selon la revendication 7, **caractérisée en ce que** dans la préparation, le dosage humain d'acide chlorogénique est de 1 à 10 mg/kg.

9. Préparation pharmaceutique pour utilisation selon la revendication 7, **caractérisée en ce que** la préparation est une préparation orale ou une injection.

10. Composition pharmaceutique pour utilisation en tant que médicament pour la prévention ou le traitement d'une douleur cancéreuse, **caractérisée en ce qu'**elle contient la préparation selon l'une quelconque des revendications 6 à 9.
